# EUROPEAN PATENT SPECIFICATION

(11) **EP 1 947 192 B1**
(45) Date of publication and mention of the grant of the patent: **03.11.2010**
(21) Application number: 06823300.6
(22) Date of filing: 10.11.2006
(51) Int. Cl.: C12Q 1/48, A61K 31/366, A61K 45/00, A61P 9/10, C12Q 1/34

(54) **PREVENTIVE/REMEDY FOR ARTERIOSCLEROTIC DISEASE AND METHOD OF EVALUATING THERAPEUTIC PROCEDURE**
MITTEL ZUR VORBEUGUNG/HEILUNG EINER ARTERIOSKLEROSEKRANKHEIT UND VERFAHREN ZUR BEWERTUNG DES THERAPIEVERFAHRENS
AGENT PRÉVENTIF/REMÈDE DESTINÉ À L'ARTÉRIOSCLÉROSE ET PROCÉDÉ PERMETTANT D'ÉVALUER UNE PROCÉDURE THÉRAPEUTIQUE

(30) Priority: 11.11.2005 JP 2005328140
(43) Date of publication of application: 23.07.2008
(73) Proprietor: Takeda Pharmaceutical Company Limited, Osaka-shi, Osaka 541-0045 (JP)
(72) Inventor: KUBO, Kazuki, Osaka-shi, Osaka 532-8686 (JP); FUSE, Hiromitsu, Osaka-shi, Osaka 532-8686 (JP); YAMAMOTO, Koji, Ibaraki 300-4293 (JP); ITASAKA, Makiko, Osaka-shi, Osaka 532-8686 (JP); NISHIMURA, Satoshi, Osaka-shi, Osaka 532-8686 (JP)
(74) Representative: Keller, Günter
(86) International application number: PCT/JP2006/322478
(87) International publication number: WO 2007/055325

(56) References cited:
- ISHII ITSUKO ET AL: "Stimulation of cholesterol release from rabbit foam cells by the action of a new inhibitor for acyl CoA:cholesterol acyltransferase (ACAT), HL-004" JOURNAL OF PHARMACOLOGY AND EXPERIMENTAL THERAPEUTICS, vol. 287, no. 1, October 1998 (1998-10), pages 115-121, XP002529998 ISSN: 0022-3565

## Description

### Technical field

The present invention relates to an evaluation method of effect of a remedy or a therapeutic procedure on an atherosclerosis-exacerbating activity of a substance (particularly, a protein) having a nature of exacerbating atherosclerosis (e.g., a pharmacological activity (e.g. an enzyme activity)) and being present in an atherosclerotic lesion, typically, ACAT (e.g., an inhibitory activity of the pharmacological activity of the target protein by an inhibitor), said remedy or therapeutic procedure directly or indirectly acting on the substance to improve the atherosclerotic lesion.

### Background Art

ACAT (acylcoenzyme A cholesterol acyltransferase) is responsible for an important role in the formation of an atherosclerotic lesion, the absorption of dietary cholesterol, lipoprotein synthesis, or the like. For this reason, an ACAT inhibitor which inhibits an activity of ACAT is expected as a preventive/remedy for atherosclerosis via a blood cholesterol level-lowering activity and foam cell formation inhibition. Thus, the development of many ACAT inhibitors has been tried up to now.

For clinically evaluating an ACAT inhibitor, the evaluation of a degree of inhibition of the enzyme activity of ACAT present in a human atherosclerotic lesion by an ACAT inhibitor is considered to be extremely important for estimating an effective dose thereof, and for correctly evaluating a clinical value of a candidate drug with a properly designed evaluation protocol.

However, since a human atherosclerotic lesion is not homogeneous and has an extremely diverse nature depending on a site thereof even in the same patient, an enzyme activity of ACAT in an atherosclerotic lesion greatly varies depending on a site, and it is considered to be extremely difficult to detect a degree of an inhibitory activity of a drug only by measuring and simply comparing enzyme activities of ACAT in atherosclerotic lesions collected from a drug-administered patient and from a drug non-administered patient. Further, since it is difficult to directly measure an amount of an ACAT protein in an atherosclerotic lesion, correction by an amount of the protein is not easy, and such evaluation has not been conventionally performed.

In addition, the evaluation of an activity of a drug on ACAT in a sample by using the sample collected from an atherosclerotic lesion has not been conventionally performed. This is considered to be difficulty in precise evaluation of an inhibitory activity of a drug due to the release of the drug from an enzyme source during a process of removing an atherosclerotic lesion and subjecting the lesion to a measurement of an ACAT activity, as well as a great variation in ACAT activity in the lesion due to the diversity of the atherosclerotic lesion.

For these reasons, conventionally, a clinical test must be initiated based on an blood lipid lowering effect of a candidate drug, and evaluation results of an examination of atherosclerotic lesion itself after a long term administration of a candidate drug in an animal model (for example, Watanabe heritable hyperlipidemic (WHHL) rabbit is widely used). Then, whether or not the effect is reflected in a human is, in fact, first revealed after considerable progression of the clinical evaluation, and a satisfactory clinical effect can not be obtained in many cases, in spite of expectation from pre-clinical data, thereby obstructing effective creation of novel drugs.

For example, Avasimibe has been recognized as an ACAT inhibitor having an anti-atherosclerotic activity of lowering a cholesterol ester in an atherosclerotic lesion in various animal models such as mouse, rabbit and hamster in pre-clinical tests (Non-Patent Documents 1 to 3). However, results of the anti-atherosclerotic activity in these pre-clinical tests are accompanied with reduction in plasma cholesterol level due to inhibition of ACAT of the liver and small intestine, and no experimental results directly showing that Avasimibe exhibits an anti-atherosclerotic activity by inhibiting ACAT in an atherosclerotic lesion have been available.

For Avasimibe, a clinical test was performed based on the results of these pre-clinical tests and, while certain effect on a blood lipid level which was thought to be based on ACAT inhibition in the liver and small intestine was observed (Non-Patent Documents 4 and 5), no effect was observed in a clinical test for examining an anti-atherosclerotic activity, and the development was ceased (Non-Patent Document 6).
Non-Patent Document 1: "Circulation, 2001, vol. 103, p. 1778 to 1786"
Non-Patent Document 2: "Atherosclerosis, 1998, vol. 137, p. 77 to 85"
Non-Patent Document 3: "Atherosclerosis, 2001, vol. 157, p. 97 to 105"
Non-Patent Document 4:" Atherosclerosis, 2001, vol. 157, p. 137 to 144"
Non-Patent Document 5:"Atherosclerosis, 2003, vol. 171, p. 273 to 279"
Non-Patent Document 6:"Circulation, 2004, vol. 110, p. 3372 to 3377"

### Disclosure of the Invention

### Problems to be solved by the Invention

Under the aforementioned circumstances, it has been desired to establish an evaluation method of a degree of an enzyme inhibitory activity on ACAT present in a human atherosclerotic lesion by an ACAT inhibitor with high precision in order to estimate clinical effect (particularly, a clinical dose) of the ACAT inhibitor with better precision. In addition, it has also been desired to establish an evaluation method of strength of atherosclerosis-exacerbating activity of not only an ACAT inhibitor, but also a general substance having a nature of exacerbating atherosclerosis, such as a protein other than ACAT, or the like present in an atherosclerotic lesion sample with high precision.

### Means for solving the problems

As compared with a case using cultured cells, an atherosclerotic lesion has complicated constituent components (versatility of cell species, necrotic focus other than cells, accumulation of lipid, fibrous substances, etc.), a composition of an atherosclerotic lesion sample is greatly different depending on an individual and a site from which the sample is collected. For this reason, since an amount of ACAT originally present in a sample is considerably different. Then, for example, even when an enzyme activity of ACAT present in a sample after administration of an ACAT inhibitor exhibited a low value, whether the low activity obtained was resulted from a small amount of ACAT originally present in the sample, or from strong inhibition of an enzyme by the ACAT inhibitor was unclear. Consequently, there was a problem that the enzyme inhibitory activity of the ACAT inhibitor can not be precisely evaluated only by an absolute value of the exhibited ACAT enzyme activity.

As solution of such a problem, for example, it is contemplated that a degree of an enzyme inhibitory activity of an ACAT inhibitor is evaluated by estimating an original ACAT enzyme activity by measuring an amount of an ACAT protein present in a collected atherosclerotic lesion sample.

However, for example, when an amount of a protein was measured by a conventional method such as a Western blot method, there was a problem that a measurement range was narrow due to the influence of contaminants derived from a tissue and a measurement error was great, thus, the intended objective could not be achieved.

On the other hand, it was contemplated that an amount of a corresponding mRNA is measured in place of direct measurement of an amount of an ACAT protein. However, the intended objective could not be achieved by this either, because stable quantitation was difficult, and an amount of an existing mRNA was not necessarily consistent with an amount of a protein.

Then, the present inventors have considered that the problem can be solved by selecting a protein whose expression amount is correlated with ACAT in an atherosclerotic lesion (hereinafter, referred to as correction marker) as an index in place of an amount of an ACAT protein, evaluating initial amounts of an ACAT protein in respective samples using an amount of a corresponding correction marker protein in place of an amount of expressed ACAT, and making correction for rectifying the difficulty in evaluation of the inhibitory activity resulted from a variation in initial amounts of ACAT protein between the respective samples by using this index. After intensive study based on this consideration, the present invention has been completed.

That is, the present invention provides:
1. A method for evaluating the inhibition of an acylcoenzyme A cholesterol acyltransferase (ACAT) enzyme activity present in an atherosclerotic lesion by an ACAT inhibitor, which comprises: determining ACAT enzyme activity in atherosclerotic lesion samples of a mammal origin collected from an administration group of the inhibitor and from a non-administration group; dividing the ACAT enzyme activity by a value corresponding to an expression amount of lysosomal acidic lipase, acidic β-galactosidase or cathepsin D as correction markers whose expression correlates with the expression of ACAT; and comparing the corrected value of one group with the corrected value of the other group.
2. The method according to claim 1, wherein the value corresponding to an expression amount of the correction markers whose expression correlates with the expression of ACAT is an enzyme activity of the correction markers.
3. The method according to claim 2, wherein the collected atherosclerotic lesion sample is of human origin.
4. A method of setting a clinical dose of an inhibitor of ACAT enzyme activity, which comprises carrying out the method of claim 3 and selecting a dose of the inhibitor having a significant difference as compared with that of a control group in the evaluation method of claim 3.

### Effect of the Invention

A similar procedure to that of the above evaluation method established for ACAT can also be applied to evaluation of an inhibitor of a pharmacological activity of a protein having a nature of exacerbating other atherosclerosis present in an atherosclerotic lesion. Further, the procedure can also be applied to evaluation of a drug or a therapeutic procedure which improves an atherosclerotic lesion by directly or indirectly acting on not only a protein but also a substance other than a protein having a nature of exacerbating atherosclerosis present in an atherosclerotic lesion. Inter alia, when it is difficult to quantitate a target protein, or when it is difficult to quantitate a substance having a nature of exacerbating atherosclerosis, the procedure becomes an advantageous evaluation method.

In the evaluation method of the present invention, a direct activity of an inhibitor on a target protein in a collected atherosclerotic lesion can be evaluated. Therefore, the effectiveness of such an inhibitor based on a direct activity on an atherosclerotic lesion can be properly evaluated and, for example, when evaluation is performed using a collected atherosclerotic lesion sample of a human patient origin, a clinical dose in an actual patient can be reasonably decided based on the effective dose in this evaluation.

### Brief Description of the Drawings

Fig. 1 is a view showing the correlation of ACAT enzyme activity and a protein concentration in a WHHL rabbit atherosclerotic lesion.
Fig. 2 is a view showing the correlation of ACAT enzyme activity and a tissue weight of a WHHL rabbit atherosclerotic lesion.
Fig. 3 is a view showing the correlation of ACAT enzyme activity and a total cholesterol content in a WHHL rabbit atherosclerotic lesion.
Fig. 4 is a view showing the correlation of ACAT enzyme activity and a free cholesterol content in a WHHL rabbit atherosclerotic lesion.
Fig. 5 is a view showing the correlation of ACAT enzyme activity and a cholesterol ester content in a WHHL rabbit atherosclerotic lesion.
Fig. 6 is a view showing the correlation of ACAT enzyme activity and BETA-GALACTOSIDASE-1 (GLB1) activity in a WHHL rabbit atherosclerotic lesion.
Fig. 7 is a view showing the correlation of ACAT enzyme activity and LYSOSOMAL ACID LIPASE (LAL) activity in a WHHL rabbit atherosclerotic lesion.
Fig. 8 is a view showing the correlation of ACAT enzyme activity and CATHEPSIN D (CTSD) in a WHHL rabbit atherosclerotic lesion.
Fig. 9 is a view showing effect of a correction with a correction marker of ACAT enzyme activity in an atherosclerotic lesion ACAT enzyme inhibiting test of a compound X.
Fig, 10 is a view showing influence on a WHHL rabbit aorta cholesterol ester content of a compound X.

### Best Mode for Performing the Invention

Hereinafter, various terms used in the evaluation method of the present invention will be illustrated in detail. However, these are not intended to be limiting. "Measurement of an activity (a pharmacological activity) of a substance (a protein) having a nature of exacerbating atherosclerosis present in a collected atherosclerotic lesion sample of a mammal origin"

### (a) Substance (protein) having nature of exacerbating atherosclerosis

The "substance having a nature of exacerbating atherosclerosis" (hereinafter, sometimes, referred to as a "target substance") to which the present invention is applied is not particularly limited, and broadly includes substances which are present or expressed in an atherosclerotic lesion, and have natures of exacerbating the atherosclerotic lesion such as extension or enlargement of the atherosclerotic lesion by exertion of their activities (pharmacological activities, etc.) [e.g., proteins, lipid (cholesterols and derivatives thereof including oxides thereof, and lipid of a class other than cholesterols and derivatives thereof including oxides thereof), substances derived from microorganisms which infect an atherosclerotic lesion, etc., particularly proteins]. Inter alia, the present invention is advantageously applied to a case where quantitation of an amount of such a target substance present in an atherosclerotic lesion is difficult.

The "protein having a nature of exacerbating atherosclerosis" to which the present invention is applied (hereinafter, sometimes, referred to as a "target protein") is not particularly limited, but broadly includes proteins which are expressed in an atherosclerotic lesion, and have natures of exacerbating the atherosclerotic lesion such as extension or enlargement of the atherosclerotic lesion or the like by exertion of their pharmacological activities. Examples of such a protein include ACAT, lipoprotein-binding type phospholipase A2, lipoxygenase, matrix metalloprotease, cathepsin, or the like.

Inter alia, the present invention is advantageously applied to a case where quantitation of an amount of such a target protein present in an atherosclerotic lesion is difficult.

### (b) Arteriosclerosis-exacerbating activity (a pharmacological activity) of a substance (a protein) having a nature of exacerbating atherosclerosis

The "atherosclerosis-exacerbating activity of a substance having a nature of exacerbating atherosclerosis" refers to an activity which is exerted by a target substance in a living body (e.g., enzyme activity, signal transmitting function, lipid accumulation, infiltration, proliferation and inflammation response of inflammatory cells, evolution and weakening of atherosclerotic lesions, etc.). For example, when a target substance is a protein, such an activity refers to its pharmacological activity.

The "pharmacological activity of a protein having a nature of exacerbating atherosclerosis" refers to a pharmacological activity which is exerted by a target protein in a living body (e.g., enzyme activity, signal transmitting function, etc.). For example, when a target protein is ACAT, such an activity refers to its enzyme activity.

### (c) Drug or therapeutic procedure directly or indirectly acting on a target substance to improve atherosclerotic lesion, and an inhibitor of a pharmacological activity of a target protein

The "drug or therapeutic procedure directly or indirectly acting on a target substance to improve an atherosclerotic lesion" refers to a drug or a therapeutic procedure which directly or indirectly acts on the aforementioned "substance having a nature of exacerbating atherosclerosis" as a target, and is not particularly limited as far as it is a drug or a therapeutic procedure having such an activity. For example, when a target substance is an enzyme protein, an enzyme inhibitor is a drug to which the present invention is applied. For example, when a target substance is lipid, a lipid lowering drug (e.g. statins, fibrates, etc.) is a drug to which the present invention is applied, and a surgical operation which reduces lipid is a therapeutic procedure.

The "inhibitor of a pharmacological activity of a target protein" refers to a drug having an inhibitory activity of a "pharmacological activity" of a target, i.e., the aforementioned "protein having a nature of exacerbating atherosclerosis", and is not particularly limited. For example, when a target protein is ACAT, an ACAT inhibitor is a drug to which the present invention is applied.

The ACAT inhibitor to which the present invention is applied is not particularly limited as far as it has an ACAT inhibitory activity. Up to now, many kinds of ACAT inhibitors have been known, and widely recognized to a person skilled in the art as those forming a one characteristic group of drugs. Examples of known ACAT inhibitors include FR-129169, CI-1011, F-1394, F-12511, T-2591, FCE-28654, K-10085, HL-004, NTE-122, FR-186054, (2E)-3-{3-[7-chloro-3-(2-{[4-fluoro-2-(trifluoromethyl)phenyl]amino}-2-oxoethyl)-6-methyyl-2-oxo-2H-chromen-4-yl]phenyl}acrylic acid, N-(1-pentyl-4,6-dimethylindolin-7-yl)-2,2-dimethylpropanamide, N-(1-octyl-5-carbocxymethtyl-9,6-dimethylindolin-7-yl)-2,2-dimethylpropanamide, and the like, but the "ACAT inhibitor" to which the present invention is applied is not limited thereto. By performing the evaluation method of the present invention, an enzyme inhibitory activity of ACAT present in an atherosclerotic lesion can be precisely evaluated.

### (d) Arteriosclerotic lesion sample

For performing the present invention, a "drug which directly or indirectly acts on a target substance to improve an atherosclerotic lesion" or an "inhibitor of a pharmacological activity of a target protein" or a control (e.g., a solvent) is administered to a mammal (e.g., rabbit, monkey, dog, human, etc.) suffering from atherosclerosis for a given period of time and, thereafter, samples collected from an atherosclerotic lesion are subjected to biochemical measurement.

The "therapeutic procedure which directly or indirectly acts on a target substance to improve an atherosclerotic lesion" is applied to a mammal (e.g. rabbit, monkey, dog, human, etc.) suffering from atherosclerosis and, after treatment for a given period of time, samples collected from an atherosclerotic lesion of a treatment group and a control (non-treated/non-processed group) is subjected to biochemical measurement.

Herein, a given period of time of administration of the "inhibitor of a pharmacological activity of a target protein" is preferably such that the influence of administration of a drug (an inhibitor) on an existing amount of a "target substance" (an expression amount of a "target protein") itself can be neglected, and it is advantageous to set the administration term, generally, for about 7 days, more preferably for about 3 days. In addition, a dose can be appropriately set by a person skilled in the art based on fundamental data such as in vitro data of a drug (an inhibitor), its blood level when administered to an animal, and the like.

Herein, an administration route of a "drug which directly or indirectly acts on a target substance to improve an atherosclerotic lesion" or an "inhibitor of a pharmacological activity of a target protein", or a control is not particularly limited, and they can be administered by appropriately selecting a route such as oral administration, intravenous administration, or the like.

In addition, when an animal other than a human is subjected to a test, atherosclerosis may be artificially made by such as high cholesterol diet, or genetically spontaneously developed. Examples of such an animal model include a WHHL rabbit.

Collection of a sample from a lesion can be performed, for example, by collecting a lesion tissue section from removed aorta. When an atherosclerotic lesion sample is collected from a human patient, it is necessary to subject a sample collected by a medically acceptable method such as (carotid endarterectomy), excision of an atherosclerotic lesion with a catheter-type equipment (e.g., Directional Coronary Atherectomy (DCA) catheter, Plaque excision system (Foxhollow)), or the like) to the evaluation.

The collected sample is subjected to a biological test after appropriate treatment such as homogenization, and the like.

### (e) Method of measuring atherosclerosis-exacerbating activity (a pharmacological activity) of a substance (a protein) having a nature of exacerbating atherosclerosis

In the present invention, a method of measuring the "atherosclerosis-exacerbating activity of a substance having a nature of exacerbating atherosclerosis" or the "pharmacological activity of a protein having a nature of exacerbating atherosclerosis" is not particularly limited, and can be measured by a known method or a similar method according to a particular action or pharmacological activity to be measured. For example, when a target protein is ACAT, a method of measuring the enzyme activity is not particularly limited, and the activity is measured by a method usually used in measuring an enzyme activity in the art. For example, when a target substance is lipid, a method of measuring an existing amount of lipid is not particular limited, and a method which is usually used in measuring an amount of lipid in the art is used.

Specifically, the measurement can be performed, for example, by collecting a given amount of an atherosclerotic lesion sample, mixing with a substrate solution containing a given amount of a substrate of ACAT (e.g. acyl-CoA such as [3H]Oleyl-CoA, etc.) labeled with a radioactive isotope in an appropriate solvent, for example, in a mixed solution of a reaction buffer (e.g. 0.19 M Tris-HCl pH 7.5, 3 mM EDTA, cholesterol 0.2 mg/ml, ESA 3 mg/ml) and water to react them at an appropriate temperature (about 37°C) for a give period of time, ceasing the reaction according to a conventional method, and measuring radioactivity of a cholesterol ester fraction after fractionation.

### "Selection of correction marker"

For performing the present invention, selection of a correction marker is of importance. Examples of requisite conditions for a correction marker include:
(a) an existing amount or an expression amount of a marker in an atherosclerotic lesion varies correlating with a value corresponding to strength of an atherosclerosis-exacerbating activity of a "target substance" present in the lesion (e.g. its existing amount) or an expression amount of a "target protein" (e.g. ACAT),
(b) a marker is conveniently used in a practical evaluation (e.g. a correction marker has an enzyme activity and quantitative evaluation can be performed using this activity as an index),
(c) a remedy (an inhibitor) or a therapeutic procedure to be evaluated does not influence on a marker, and the like.

Specifically, the selection of a preferred correction marker can be performed, for example, by narrowing candidates by the following procedure (provided that, this is not limiting).
1) By utilizing a gene expression database, a gene whose expression amount correlates with a "target protein" (when the target protein is ACAT, for example, ACAT-1 which is a subtype) present in an atherosclerotic lesion is selected. As a rule, it is advantageous to narrow correction marker candidates in an order from higher correlation.
2) A candidate having an enzyme activity is selected as a correction marker from proteins encoded by the gene.
3) From a viewpoint of simplicity and specificity of a measuring method, a highly convenient candidate is selected as a correction marker. For example, it is advantageous to select a candidate having such an enzyme activity that a substrate whose enzyme reaction can be monitored by a general detection system such as colorimetry and fluorescence is available. In addition, it is preferable to select a candidate so that measuring conditions of a correction marker do not influence on an evaluation of a pharmacological activity of a target protein or quantitation of other correction markers. Conversely, it is also preferable to select a candidate so that the measuring conditions of a pharmacological activity of a target protein do not influence on quantitation of a correction marker.
4) A candidate is selected so that an expression amount of a correction marker in an atherosclerotic lesion has correlation with a "pharmacological activity of a target protein" (e.g. ACAT enzyme activity) in an atherosclerotic lesion derived from a mammal (e.g. rabbit, monkey, dog, human etc.).
5) From the aforementioned viewpoint, candidates are narrowed, and a correction marker to be used is selected.

When a target protein is ACAT, examples of thus selected preferable correction marker include a protein such as lysosomal acidic lipase (LAL), acidic β galactosidase (GLB1), cathepsin D (CTSD), and the like.

In case that a "target substance" is other than a protein, a correction marker can also be selected by a similar method. A correction marker may be other than a protein as far as it satisfies the conditions for a correction marker described hereinafter, and is not particularly limited.

### "Correction by a correction marker"

Correction by a correction marker in the present invention is performed for complementing the following point.

When a "target substance" or a "pharmacological activity of a target protein" (e.g., ACAT enzyme activity) in a atherosclerotic lesion sample is directly compared and studied, an atherosclerotic lesion improving activity (enzyme inhibitory activity) by a drug or a therapeutic procedure (ACAT inhibitor) can not be precisely evaluated because there is a great variation in an existing amount (expression amount) of an originally existing "target substance" ("target protein" (e.g. ACAT)) between samples.

Herein, a "value corresponding to an amount (expression amount) in an atherosclerotic lesion sample of a correction marker" refers to a value which quantitatively varies correlating with an expression amount of the correction marker, by which a degree of expression of the correction marker can be detected by measuring the value.

For example, when a correction marker used has a function as an enzyme, a degree of an enzyme activity can be used as the value corresponding to an expression amount of the correction marker in the atherosclerotic lesion sample. Such an enzyme activity can be measured by a conventional method (e.g. a measuring method described in Examples hereinafter) depending on a particular enzyme activity, provided that a correction marker is not limited to one whose index is an enzyme activity. For example, when an expression amount itself of a correction marker can be measured, the value measured can be used as a "value corresponding to an expression amount in an atherosclerotic lesion sample of a correction marker". Alternatively, there is a case where an amount of an mRNA encoding a correction marker can be used.

Correction is performed, for example, as follows.

A "pharmacological activity of a target protein" (e.g. ACAT enzyme activity) measured with respect to each atherosclerotic lesion sample collected from a mammal of an inhibitor administration group is divided by a "value corresponding to an expression amount in an atherosclerotic lesion sample of a correction marker" (e.g. an enzyme activity of a correction marker) to calculate a pharmacological activity of a target protein corrected with a correction marker of each atherosclerotic lesion sample ("post-inhibitor administration corrected target protein pharmacological activity").

On the other hand, with respect to an atherosclerotic lesion samples collected from a mammal of an inhibitor non-administration group (a control group), corrected target protein pharmacological activities are similarly calculated, and an average thereof is adopted as a "base-corrected target protein pharmacological activity" corrected by the correction marker.

The already calculated "post-inhibitor administration corrected target protein pharmacological activity" of each atherosclerotic lesion sample is converted into a proportion by taking a "base corrected target protein pharmacological activity" as 100% to calculate an "post-inhibitor administration corrected target protein pharmacological activity ratio" (% of Control).

A "post-inhibitor administration corrected target protein pharmacological activity ratio" obtained with respect to each atherosclerotic lesion sample is statistically treated by a conventional method (e.g. Student t-test, variance analysis, etc.) to evaluate an inhibitory degree of a pharmacological activity of a target protein by an inhibitor.

Since a correction marker selected according to the aforementioned method is expressed correlating with an amount of a target protein expressed in a sample irrespective of its origin, an inhibitory activity of a pharmacological activity of a target protein by an inhibitor can be quantitatively compared and studied irrespective of a sample origin by converting a pharmacological activity of a target protein measured into the "post-inhibitor administration corrected target protein pharmacological activity ratio" as described above.

Thereby, a variance in amounts of target proteins between samples can be corrected to precisely evaluate strength and weakness of an activity of an inhibitor.

Similarly, a "value corresponding to strength of atherosclerosis-exacerbating activity of a target substance" (e.g., an existing amount of target substance) measured with respect to each atherosclerotic lesion sample collected from a mammal of an administration- or treatment-group of a drug or a therapy which improves an atherosclerotic lesion is corrected (e.g., divided) with a "value corresponding to an amount of a correction marker in an atherosclerotic lesion sample " to calculate an atherosclerosis-exacerbating activity of a target substance corrected with a correction marker of each atherosclerotic lesion sample.

### "Setting of clinical dose"

When the evaluation by the present invention is applied to an atherosclerotic lesion of a human origin, a clinical dose of a human can be set.

In the evaluation by the present invention, a clinical dose can be set by selecting a dose of an inhibitor which inhibits a pharmacological activity of a target protein with a significant difference to a control group. For selecting doses, clinical doses having certain range can be also set by selecting two or more doses of an inhibitor which inhibits a pharmacological activity of a target protein with a significant difference to a control group.

When a clinical dose is set, an inhibitory activity of a pharmacological activity of a target protein by an inhibitor can also be evaluated according to the present invention using a plurality of correction markers, and respective evaluation results are statistically treated as a whole, thereby determining a clinical dose according to the aforementioned procedure.

An effective dose of an "inhibitor of a pharmacological activity of a target protein" (clinical dose in case of medical use) to be evaluated can be determined by the evaluation method of the present invention and, based on this, for example, a dose per unit dosage of an inhibitor for a patient suffering from an atherosclerotic disease can be determined.

Therefore, a preventive/remedy for an atherosclerotic disease can be obtained by producing a pharmaceutical composition comprising the aforementioned clinical dose or a larger dose. Specifically, an inhibitor of a pharmacological activity of a target protein can be administered in the form of a pharmaceutical composition which is prepared so as to contain the aforementioned clinical dose (or larger) of the inhibitor per a unit dosage form.

Herein, examples of the "atherosclerotic disease" include ischemic heart diseases such as myocardial infarction, angina pectoris, and the like, cerebrovascular diseases such as cerebral hemorrhage, cerebral infarction and the like, aortic aneurysm of great vessel, peripheral artery disease, , and the like.

A pharmaceutical composition can be formulated in the form of a preparation according to a conventional method. Usually, such a preparation can be produced by mixing and/or kneading an active ingredient with one or more additives such as excipients, diluents, carriers, and the like. The term "parenteral" used herein includes subcutaneous injection, intravenous injection, intramuscular injection, intraperitoneal injection, drip infusion, and the like. An injectable preparation, for example, an aseptic injectable aqueous suspension or oily suspension can be prepared by a method known in the art using a suitable dispersant or a wetting agent and a suspending agent. Further, an aseptic injectable preparation may be, for example, that an aseptic injectable solution or suspension in a diluent or a solvent, which is nontoxic and can be administered parenterally, such as an aqueous solution. Examples of an allowable vehicle or solvent include water, Ringer' solution and an isotonic saline. Furthermore, usually, aseptic nonvolatile oil can also be used as a solvent or a suspending solvent. For this purpose, any nonvolatile oil and fatty acids can be used, and a natural or synthetic or semi-synthetic fatty oil or fatty acids, and natural or synthetic or semi-synthetic or mono- or di- or triglycerides may be included.

A suppository for rectal administration can be produced by mixing a drug with a suitable non-irritable adjuvant, for example, an adjuvant which is solid at ordinary temperature, but is liquid at a temperature of the intestinal tract, and is melted in the rectum to release a drug, such as cocoa butter and polyethylene glycols.

Alternatively, it is effective to combine with a suitable base (e.g. polymers of butyric acid, polymers of glycolic acid, copolymers of butyric acid-glycolic acid, mixture of polymer(s) of butyric acid and polymer(s) of glycolic acid, polyglycerol fatty acid esters, etc.) to prepare a sustained-release preparation.

Examples of a solid dosage form for oral administration include powders, granules, tablets (including oral disintegrating tablets), pills, capsules, films (including oral disintegrating films), and the like. A preparation in such a dosage form can be produced by mixing and/or kneading an active ingredient compound with at least one additive, for example, sucrose, lactose, cellulose sugar, mannitol (D-mannitol), maltitol, dextran, starches (e.g. corn starch), microcrystalline cellulose, agar, alginate, chitin, chitosan, pectin, tragacanth gum, gum arabic, gelatin, collagen, casein, albumin, synthetic or semi-synthetic polymers or glycerides. Usually, such a dosage form can contain further additive(s), and examples thereof include inert diluents, lubricants such as magnesium stearate, preservatives such as parabens and sorbic acid, antioxidants such as ascorbic acid, α-tocopherol and cysteine, disintegrating agents (e.g., croscarmellose sodium), binders (e.g., hydroxypropylcellulose), thickeners, buffering agents, sweeteners, flavors, perfumes, and the like. Further, tablets and pills can be subjected to enteric-coating. Examples of liquid preparations for oral administration include pharmaceutical acceptable emulsions, syrup, elixirs, suspensions, and solutions, and they may contain inert diluents which are normally used in the art, for example, water and, if necessary, additives. These oral liquid preparations can be produced according to a conventional method by mixing an active ingredient compound and an inert diluent and, if necessary, other additives.

Hereinafter, the present invention will be illustrated in more detail by way of Examples, but the present invention is not limited thereto.

### Example 1

### "Test method"

### 1. Selection of correction marker using gene expression database

From a database concerning gene expression in 121 cases of human atherosclerotic lesions (manufactured by Gene logic), genes whose expression amounts were correlated with ACAT-1, an ACAT subtype, present in an atherosclerotic lesion were selected, and a protein corresponding to a gene with a high correlation coefficient, and having an enzyme activity was selected as a correction marker.

### 2. Preparation of atherosclerotic lesion sample

"Administration of ACAT inhibitor, and collection of artery "

Using a rabbit having an atherosclerotic lesion in the aorta due to rearing with a high cholesterol diet, or due to hereditary hyperlipemia, etc., an ACAT inhibitor or a medium was orally administered once a day for 3 days. About two hours after final administration, the rabbit was anesthetized and killed by exsanguination, and the aorta was removed, frozen and stored. After thawing the frozen-stored aorta, a tissue section of an atherosclerotic lesion residing site was corrected using a biopsy trephine. Alternatively, a tissue section of an atherosclerotic lesion which had been corrected using a biopsy trephine at removal of the aorta, frozen and stored was thawed, and was subjected to an experiment hereinafter.

### "Preparation of atherosclerotic lesion homogenate"

A tissue specimen of an atherosclerotic lesion was minced with scissors, a given amount of STE buffer (250 mM sucrose, 50 mM Tris-HCl pH 7.0, 1 mM EDTA-2Na, Leupeptin 2 µg/ml) was added thereto, homogenized, and centrifuged (4000 rpm, 5 min) to remove the tissue residue to obtain a homogenate, which was used as an "atherosclerotic lesion sample" for a biochemical measurement.

### 3. Measurement of various biological activities

### (1) Measurement of ACAT enzyme activity

An ACAT reaction was measured by mixing a reaction buffer (0.19 M Tris-HCl pH 7.5, 3 mM EDTA, cholesterol 0.2 mg/ml, BSA 3 mg/ml), the aforementioned "atherosclerotic lesion sample", and a substrate solution ([3H] Oleoyl-CoA), reacting the mixture with stirring at 37°C for 30 minutes, fractionating a lipid fraction by thin layer chromatography, and measuring radioactivity (DPM) incorporated into a cholesteryl ester fraction with a liquid scintillation counter. Radioactivity of a blank sample (STE buffer instead of the homogenate was added) was also measured, and a value obtained by subtracting radioactivity of the blank from radioactivity of each sample was adopted as the ACAT enzyme activity.

### (2) Measurement of protein concentration

A protein concentration of the homogenate was measured using a BCA protein assay kit (PIERCE) by employing bovine serum albumin having a known concentration as a standard.

### (3) Measurement of cholesterol content

Total cholesterol, free cholesterol, and cholesteryl ester in the homogenate were measured by the following procedure. According to a conventional method, total lipid was extracted and recovered from the homogenate, a total cholesterol concentration and a free cholesterol concentration were measured using Cholesterol E Test Wako (Wako Pure Chemical Industries, Ltd.), and free Cholesterol E Test Wako (Wako Pure Chemical Industries, Ltd.) to calculate a total cholesterol content and a free cholesterol content in the homogenate. A value obtained by subtracting the free cholesterol content from the total cholesterol content was adopted as a cholesteryl ester content.

### (4) Measurement of lysosomal acidic lipase (LAL) activity

The measurement was performed by modifying a part of the method reported by S. Zhang at al. (Biochemical Journal, vol. 3 48, P. 621 to 632). Namely, to a microplate for measuring fluorescence were added 100 µL of a buffer (0.4 M sodium acetate pH 5.5), 50 µL of water, and 25 µL of an "arteriosclerotic lesion sample" homogenate, this was kept at 37°C, 25 µL of a 4-methylumbellyferyl oleate solution as a substrate was added to each well to perform a reaction at 37°C for 30 minutes, and fluorescence of Ex 355/Em 460 was measured with a fluorescence plate reader. In place of the "atherosclerotic lesion sample" homogenate, a buffer for preparing a homogenate was added to perform a reaction, and fluorescence was measured. This was used as a blank, and a value obtained by subtracting the blank from the fluorescence of a sample was adopted as the activity of the sample.

### (5) Measurement of acidic β galactosidase (GLB1) activity

The measurement was performed by modifying a part of the method reported by S. Scheriff et al. (Journal of Biological Chemistry, 1995, vol. 270, P. 27766 to 27772). Namely, to a microplate for measuring fluorescence were dispensed 100 µL of a buffer (80 mM citrate-160 mM sodium phosphate buffer (pH 4.4)), 50 µL of water, and 25 µL of an "atherosclerotic lesion sample" homogenate, this was sealed, and kept at 37°C, 25 µL of a 4-methyllumbellyferyl-β-galactopyranoside solution as a substrate was added to each well to perform a reaction at 37°C for 30 minutes, and fluorescence of Ex 355/Em 460 was measured with a fluorescence plate reader. In place of the "atherosclerotic lesion sample" homogenate, a buffer for preparing a homogenate, a STE buffer was added to perform a reaction, and fluorescence was measured. This was used as a blank, and a value obtained by subtracting the blank from the fluorescence of a sample was adopted as the activity of the sample.

### (6) Measurement of cathepsin D (CTSD) activity

To a microplate for measuring fluorescence were dispensed 50 µL of an "atherosclerotic lesion sample" homogenate, and 50 µL of an Ac-Gly-Lys-Pro-Ile-Leu-Phe-Phe-Arg-Leu-Lys(Dnp)-D-Arg-NH2 solution as a substrate to perform a reaction at 37°C for 30 minutes, and fluorescence of Ex 320/Em 405 was measured with a fluorescence plate reader. In place of the "atherosclerotic lesion sample" homogenate, a buffer for preparing a homogenate was added to perform a reaction, and fluorescence was measured. This was used as a blank, and a value obtained by subtracting the blank from the fluorescence of a sample was adopted as the activity of the sample.

### 4. Correction of ACAT enzyme activity

The ACAT enzyme activity calculated on a homogenate for each atherosclerotic lesion sample was divided by a protein amount, a cholesterol amount, lysosomal acidic lipase (LAL) activity, acidic β galactosidase (GLB1) activity, or cathepsin D (CTSD) activity to calculate a corrected ACAT enzyme activity with each correction marker. Further, taking an average of the corrected ACAT enzyme activity of a drug-untreated rabbit sample (control group) to be 100%, a ratio of corrected ACAT enzyme of a drug-treated rabbit sample (% of Control) was obtained.

### "Test result"

### 1. Selection of enzyme whose expression in atherosclerotic lesion is correlated with ACAT-1

Each activity measurement system was set by selecting an enzyme having a high correlation coefficient and an enzyme activity, and further, in view of convenience for construction of an activity measurement system, selecting lysosomal acidic lipase (LAL), acidic β galactosidase (GLB1), and cathepsin D (CTSD) (Table 1).

**Table 1**

| Selection of gene whose expression amount in atherosclerotic lesion is correlated with ACTA-1 | | |
|---|---|---|
| Gene | Abbreviation | Correlation coefficient |
| STEROL I-ACYLTRANSFERASE 1 (ACAT-1) | SOAT1 | 1.000 |
| ACID CERAMIDASE | ASAH1 | 0.708 |
| CATHEPSIN S | CTSS | 0.699 |
| BRANCHED-CHAIN AMINO TRANSFERASE 1 | BCAT1 | 0.684 |
| SPERMIDINE/SPERMINE N(1)-ACETYL TRANSFERASE | SAT | 0.680 |
| ARACHIDONATE 5-LIPOXYGENASE | ALOX5 | 0.678 |
| CATHEPSIN L | CTSL | 0.677 |
| PHSPHOLIPASE A2, GROUP VII | PLA2G7 | 0.677 |
| BERA-GALACTOSIDASE-1 | GLB1 | 0.668 |
| FRUCTOSE-1, 6-BISPHOSPHATASE 1 | FBP1 | 0.666 |
| PALMITOYL-PROTEIN THIOESTERASE 1 | PPT1 | 0.658 |
| LACTAMASE, BETA | LACTB | 0.656 |
| CATHEPSIN D | CTSD | 0.644 |
| PHOSPHOGLYCERATE KINASE | PGK1 | 0.644 |
| GLUTATHIONE PEROXIDASE | GPX1 | 0.642 |
| ALPHA-GALACTOSIDASE A | GLA | 0.632 |
| LYSOSOMAL ACID LIPASE | LAL | 0.631 |
| SPHINGOSINE-1-PHOSPHATE LYASE1 | SGPLA1 | 0.623 |

### 2. Correlation between ACAT enzyme activity in WHHL rabbit atherosclerotic lesion and various indices

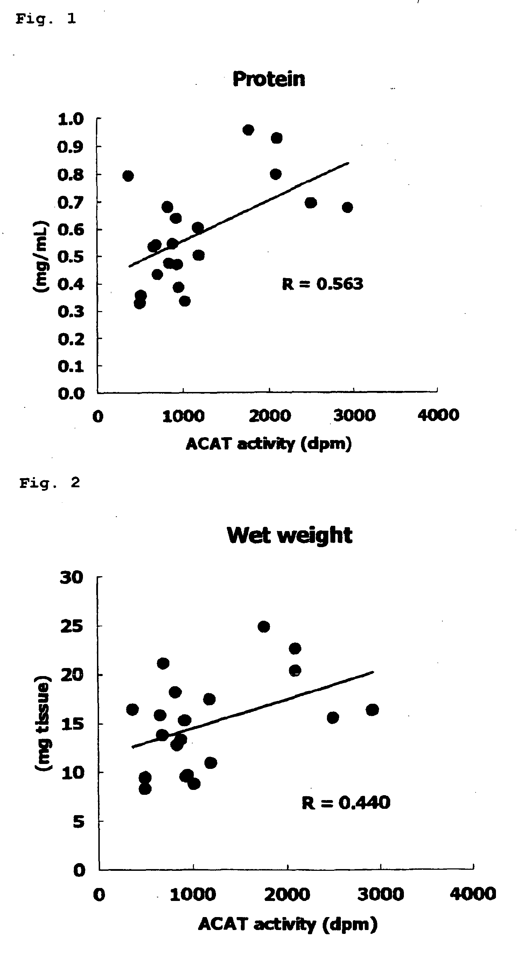

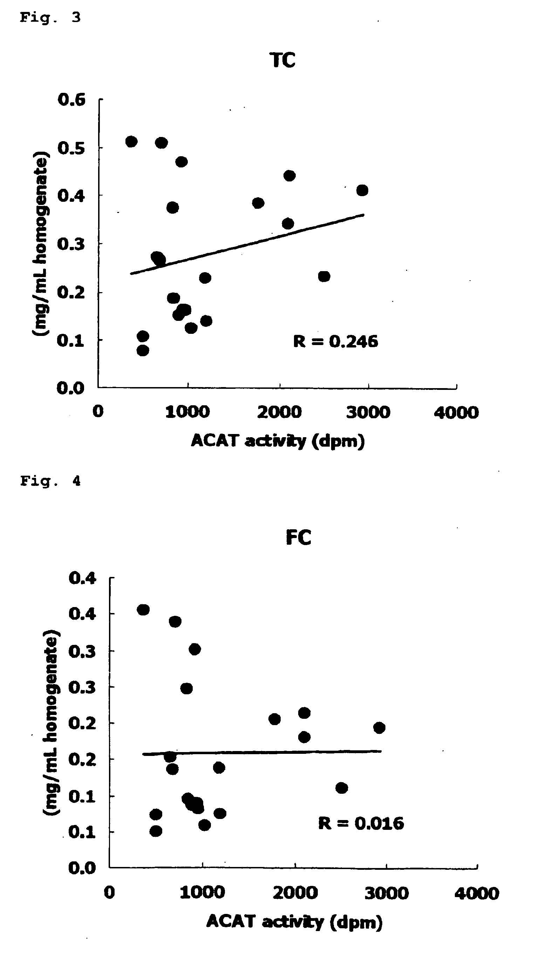

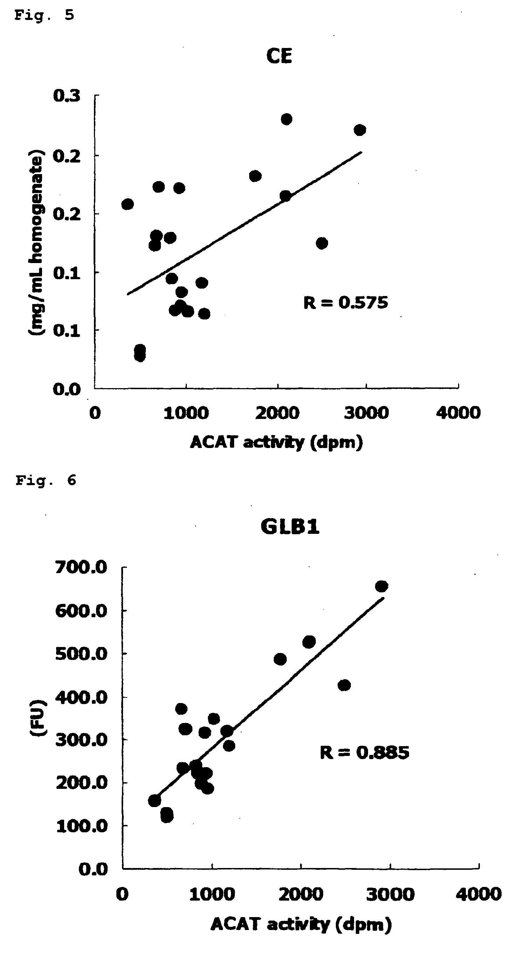

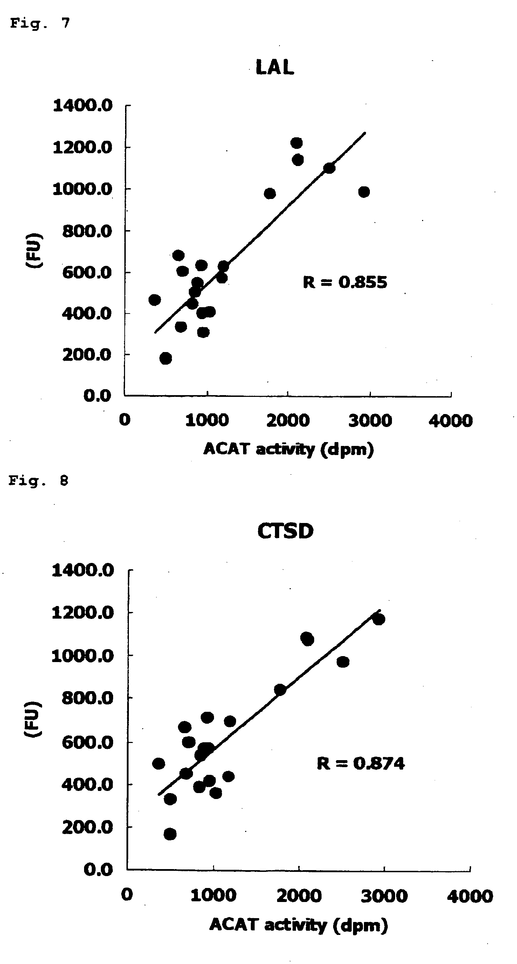

Regarding each of 20 samples of atherosclerotic lesion site from WHHL rabbit, a tissue homogenate was prepared, the ACAT enzyme activity and various indices (the aforementioned three kinds of correction markers, as well as a protein concentration (protein), a lesion tissue weight (wet weight), a total cholesterol content (TC), a free cholesterol content (FC), and a cholesteryl ester content (CE)) were measured, and correlation with the ACAT enzyme activity was examined (Figs. 1 to 8).

As a result, the ACAT enzyme activity had high correlation with the GLB1 activity, the LAL activity and the CTSD activity as compared with the protein concentration (protein), the lesion tissue weight (wet weight), the total cholesterol content (TC), the free cholesterol content (FC), and the cholesteryl ester content (CE). Thus, it was demonstrated that three kinds of correction markers having high correlation with ACAT-1 in terms of a gene expression level in human atherosclerotic lesions actually had high correlation also in terms of an enzyme activity level in rabbit atherosclerotic lesions.

### 3. Effect of correction with correction markers of ACAT enzyme activity in atherosclerotic lesion ACAT enzyme inhibition test with ACAT inhibitor

### (1) Test compound

As an ACAT inhibitor, the following compound (hereinafter, referred to as compound X) was used to perform a test.
Monopotassium bis((2E)-3-{3-[7-chloro-3-(2-{[4-fluoro-2-(trifluoromethyl)phenyl]amino}-2-oxoethyl)-6-methyl-2-oxo-2H-chromen-4-yl]phenyl}acrylate) trihydrate

This compound was synthesized as follows.

This test compound X is disclosed in International Patent Application PCT/JP2005/003838.

A mixture of sodium (2E)-3-{3-[7-chloro-3-(2-{[4-fluoro-2-(trifluoromethyl)phenyl]amino}-2-oxoethyl)-6-methyl-2-oxo-2H-chromen-4-yl]phenyl}acrylate (5.0 g), ethanol (45 ml) and water (9 ml) was heated to 60°C to prepare a solution. After removing impurities in the solution by filtration, the solution was washed with warmed ethanol/water (5/1, 6 ml). The filtrate and the washing were combined and an aqueous solution (10 ml) of potassium chloride (0.524 g) was added dropwise thereto. After stirring at 60°C for 3 hours, the mixture was further stirred at 25°C for 1 hour. Crystals formed were collected by filtration, and washed successively with ethanol/water (1/1, 10 ml) and water (10 ml x 3) to obtain the compound X (4.47 g, yield: 89.8%) as colorless crystals. A crystallization degree was 66%.
¹H-NMR (300 MHz, DMSO-d₆) δ (ppm): 2.17 (3H, s), 3.32 (2H, br), 6.47 (1H, d, J=16 Hz), 6.87 (1H, s), 7.21-7.23 (1H, m), 7.32-7.54 (6H, m), 7.66 (2H, s), 9.55 (1H, brs)

### (2) Test method

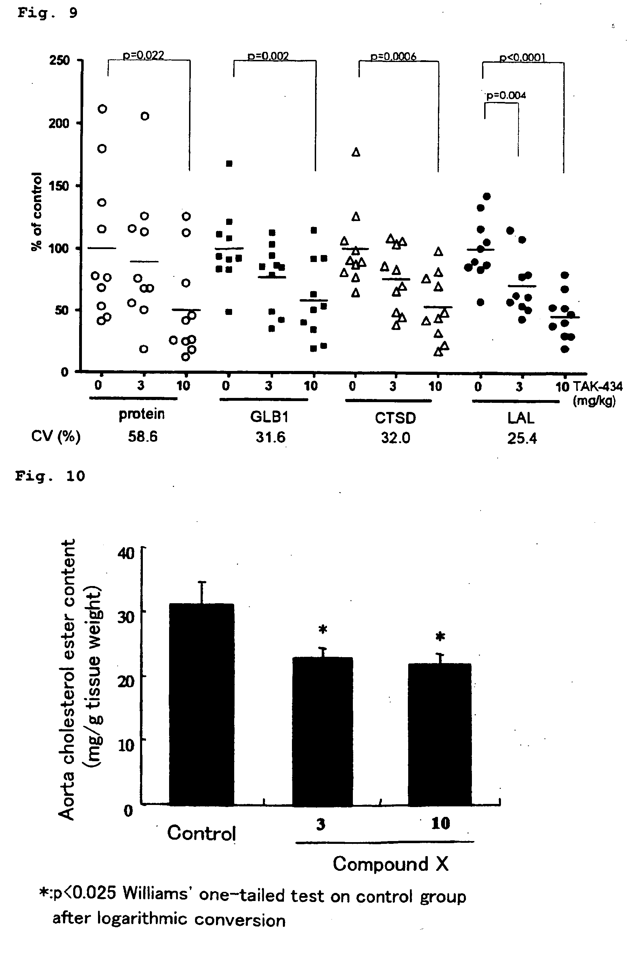

Thirty WHHL rabbits were divided into three groups of each 10 animals, i.e., a control group (medium (0.5% methylcellulose) administration), a 3 mg/kg ACAT inhibitor (compound X) administration group, and a 10 mg/kg ACAT inhibitor (compound X) administration group, and atherosclerotic lesion samples were collected after administration once a day for 3 days. ACAT enzyme activity and correction indices (protein concentration, lysosomal acidic lipase (LAL) activity, acidic β galactosidase (GLB1) activity, cathepsin D (CTSD) activity) of each atherosclerotic lesion tissue homogenate were measured. The ACAT enzyme activity corrected with each index is shown in Fig. 9.

In the control group, all of LAL, GLB1, and CTSD showed high correlation with the ACAT enzyme activity as compared with the protein concentration. In addition, while the ACAT enzyme activity varied greatly by protein concentration correction, the variance of ACAT enzyme activity was small due to correction with three enzyme (LAL, GLB1, CTSD) activities. Then, it became possible to precisely evaluate the activity strength of the inhibitor.

As a result, while the ACAT enzyme activity of the 10 mg/kg compound X administered group only showed inhibition at a p value of 0.022 in case of the protein concentration correction, remarkable inhibition at a p value of 0.002 or less could be detected in the 10 mg/kg compound X administered group in case of the GLB1 and CTSD correction and further, in case of the LAL correction, significant inhibition could be detected even in the 3 mg/kg compound X administration group. Thus, the inhibition activity could be evaluated at a better precision.

### 4. Anti-atherosclerotic activity test of compound X in WHHL rabbit

Regarding 3 and 10 mg/kg compound X administration groups wherein the inhibitory activity was observed in the aforementioned atherosclerotic lesion ACAT enzyme inhibition test using the WHHL rabbit, anti-atherosclerotic activity by long-term administration was examined.

### (Test method)

Using 7-month-old WHHL rabbits, the compound X (3, 10 mg/kg/day) was orally administered once a day for 12 weeks and influence on atherosclerosis was examined. Before drug administration, aortic atherosclerosis was tested by intravascular ultrasound (IVUS), and WHHL rabbits were grouped based on the aortic atherosclerosis, plasma cholesterol level and sex.

Before initiation of administration, and 1, 2, 4, 6, 8, 10, 12 weeks from administration, blood samples were collected, a plasma total cholesterol level of each sample was measured using a biochemical automatic analysis apparatus and a plasma total cholesterol exposure amount during the administration period was obtained by a trapezoid formula. After administration, the thoracic aorta was removed, and a cholesterol ester content as an index of atherosclerosis was measured.

### (Result)

Compound X at the doses of 3 and 10 mg/kg showed only a reduction tendency relative to a plasma total cholesterol exposure amount during the administration period (Table 2), but significantly reduced a cholesterol ester content of the thoracic aorta (Fig. 10).

**Table 2**

| Influence of compound X on plasma cholesterol in WHHL rabbit | |
|---|---|
| | Plasma total cholesterol exposure amount during administration period (mg·week/dL) |
| Control | 6194 ± 511 |
| Compound × 3 mg/kg | 5356 ± 410 |
| Compound × 10 mg/kg | 4957 ± 490 |

| | |
|---|---|
| Average ± standard error. No significant difference by Williams' one-tailed test compared to a control group. | |

### 5. Discussion

It was possible to evaluate more clearly by the administration test for 3 days (see the aforementioned test 3) that the ACAT inhibitor, i.e., the compound X inhibited the atherosclerotic lesion ACAT activity of the WHHL rabbit dose-dependently by utilizing the correction marker.

On the other hand, the compound X exerted the clear anti-atherosclerotic activity in the animal model test by long-term treatment at doses of 3 and 10 mg/kg at which the atherosclerotic lesion ACAT was inhibited (see the aforementioned test 4).

From these results, regarding an ACAT inhibitor, it was considered that, by examining the inhibitory activity of ACAT pharmacological activity present in an atherosclerotic lesion by short term administration, an effective dose for the anti-atherosclerotic activity requiring for long term treatment (in the same animal as that from which an atherosclerotic lesion was collected) could be estimated.

This can be considered to be applied to not only WHHL rabbits but also a human. Thus, it is considered to be possible to estimate an effective dose of an anti-atherosclerotic activity test and an atherosclerotic disease prevention test requiring a long term, that is, a clinical dose, by examining a human-derived atherosclerotic lesion ACAT inhibitory activity by a short term ACAT inhibitor treatment. In addition, assuming that an ACAT inhibitor exerts the effect at the same blood drug level in a rabbit as well as a human, it is considered to be possible to estimate an effective dose in a human using a blood drug level in a clinical test as an index, by examining a blood drug level at an effective dose in a WHHL rabbit.

### Industrial applicability

The present invention relates to an evaluation method of an inhibitory activity by an inhibitor of a pharmacological activity of a target protein, relative to the pharmacological activity (e.g. enzyme activity) of a protein having a nature of exacerbating atherosclerosis present in an atherosclerotic lesion, typically ACAT. The present invention has an advantage that a clinical dose at a clinical test stage and post-marketing can be set, and is also advantageous in selecting a candidate compound at a research stage and pre-clinical test stage, and re-setting an effective dose for a patient at a stage of administration of the inhibitor to a patient at a medical field.

In addition, the present invention generally relates to an evaluation method of a drug or a therapeutic procedure for directly or indirectly acting on a substance having a nature of exacerbating atherosclerosis present in an atherosclerotic lesion to improve the atherosclerotic lesion, relative to the atherosclerosis-exacerbating activity of the substance.

## Claims

1. A method for evaluating the inhibition of an acylcoenzyme A cholesterol acyltransferase (ACAT) enzyme activity present in an atherosclerotic lesion by an ACAT inhibitor, which comprises: determining ACAT enzyme activity in atherosclerotic lesion samples of a mammal origin collected from an administration group of the inhibitor and from a non-administration group; dividing the ACAT enzyme activity by a value corresponding to an expression amount of lysosomal acidic lipase, acidic β-galactosidase or cathepsin D as correction markers whose expression correlates with the expression of ACAT; and comparing the corrected value of one group with the corrected value of the other group.

2. The method according to claim 1, wherein the value corresponding to an expression amount of the correction markers whose expression correlates with the expression of ACAT is an enzyme activity of the correction markers.

3. The method according to claim 2, wherein the collected atherosclerotic lesion sample is of human origin.

4. A method of setting a clinical dose of an inhibitor of ACAT enzyme activity, which comprises carrying out the method of claim 3 and selecting a dose of the inhibitor having a significant difference as compared with that of a control group in the evaluation method of claim 3.

## Patentansprüche

1. Verfahren zur Bewertung der Inhibierung einer Acyl-Co-Enzym-A-Cholesterin-Acyltransferase- (ACAT-) -Enzymaktivität, die in einer atherosklerotischen Läsion vorliegt, durch einen ACAT-Inhibitor, welches: das, Bestimmen der ACAT-Enzymaktivität in Proben einer atherosklerotischen Läsion von einem Säuger, die von einer Verabreichungsgruppe des Inhibitors und von einer Nicht-Verabreichungsgruppe genommen wurden; das Dividieren der ACAT-Enzymaktivität durch einen Wert, der einer Expressionsmenge einer lysosomalen, sauren Lipase, saurer β-Galactosidase oder Cathepsin D als Korrekturmarker entspricht, deren Expression mit der Expression von ACAT korreliert; und das Vergleichen des korrigierten Wertes einer Gruppe mit dem korrigierten Wert der anderen Gruppe umfasst.

2. Verfahren nach Anspruch 1, wobei der Wert, der einer Expressionsmenge der Korrekturmarker entspricht, deren Expression mit der Expression von ACAT korreliert, die Enzymaktivität der Korrekturmarker ist.

3. Verfahren nach Anspruch 2, wobei die gesammelten Proben einer atherosklerotischen Läsion menschlichen Ursprungs sind.

4. Verfahren zum Festlegen einer klinischen Dosis eines Inhibitors der ACAT-Enzymaktivität, welches das Durchführen des Verfahrens von Anspruch 3 und das Auswählen einer Dosis des Inhibitors mit einem signifikanten Unterschied im Vergleich zu dem einer Kontrollgruppe in dem Bewertungsverfahren von Anspruch 3 umfasst.

## Revendications

1. Procédé d'évaluation de l'inhibition d'une activité de l'enzyme acyl-coenzyme A cholestérol acyltransférase (ACAT) présente dans une lésion athérosclérotique par un inhibiteur de l'ACAT, qui comprend : déterminer l'activité de l'enzyme ACAT dans des échantillons de lésion athérosclérotique d'une origine mammifère, collectés dans un groupe d'administration de l'inhibiteur et dans un groupe de non administration ; diviser l'activité de l'enzyme ACAT par une valeur correspondant à une quantité d'expression de la lipase acide lysosomale, de la β-galactosidase acide ou de la cathépsine D en tant que marqueurs de correction dont l'expression est corrélée à l'expression de l'ACAT ; et comparer la valeur corrigée d'un groupe à la valeur corrigée de l'autre groupe.

2. Procédé selon la revendication 1, dans lequel la valeur correspondant à une quantité d'expression des marqueurs de correction dont l'expression est corrélée à l'expression de l'ACAT est une activité enzymatique des marqueurs de correction.

3. Procédé selon la revendication 2, dans lequel l'échantillon de lésion athérosclérotique collecté est d'origine humaine.

4. Procédé de définition d'une dose clinique d'un inhibiteur de l'activité de l'enzyme ACAT, qui comprend réaliser le procédé selon la revendication 3 et choisir une dose de l'inhibiteur significativement différente de celle d'un groupe témoin dans le procédé d'évaluation selon la revendication 3.
